Europäisches Patentamt

European Patent Office

Office européen des brevets

(11) Publication number: 0 358 336
A1

## (12) EUROPEAN PATENT APPLICATION

(21) Application number: 89308077.0

(22) Date of filing: 09.08.89

(51) Int. Cl.⁵: A61B 17/36 , A61N 1/32

(30) Priority: 11.08.88 GB 8819114

(43) Date of publication of application:
14.03.90 Bulletin 90/11

(84) Designated Contracting States:
AT BE CH DE ES FR GB GR IT LI LU NL SE

(71) Applicant: Boyd, Edward George Charles
Arthur, Dr.
39, Purley Road
South Croydon Surrey CR2 6EZ(GB)

Applicant: Holt, Phyllis Marie, Dr.
16, Iris Close
Walderslade Chatham Kent ME5 9QD(GB)

(72) Inventor: Boyd, Edward George Charles
Arthur, Dr.
39, Purley Road
South Croydon Surrey CR2 6EZ(GB)
Inventor: Holt, Phyllis Marie, Dr.
16, Iris Close
Walderslade Chatham Kent ME5 9QD(GB)

(74) Representative: Brown, John David et al
FORRESTER & BOEHMERT
Widenmayerstrasse 4/l
D-8000 München 22(DE)

(54) Apparatus for effecting controlled tissue destruction.

(57) Apparatus for effecting controlled destruction of limited areas of living tissue e.g. his bundle ablation, by applying electrical pulses of high current to the tissue, having an electrical pulse generator (5), a probe with an electrode (4) to be placed close to or in the tissue area to be treated and a second electrode (6) for placement in contact with the patient (2) to complete the electrical circuit. The pulse generator (5) is adapted to produce a series of electrical pulses having a voltage of between 500 and 3600 Volts and a duration of between 5 and 500 microseconds.

## Apparatus for effecting controlled tissue destruction

This invention relates to apparatus for effecting controlled destruction of limited areas of living tissue by applying electrical pulses of high current to the tissue, having an electrical pulse generator, a probe with an electrode to be placed close to or in the tissue area to be treated and a second electrode for placement in contact with the patient to complete the electrical circuit. The second electrode may be a large area skin electrode to be connected from the outside to the patient or it may be a small electrode similar to the first electrode. In this latter configuration the second electrode may also be used to produce a limited area of tissue damage (so-called bipolar technique).

In various surgical operations limited areas of living tissue are intentionally destroyed to stop further growth of the tissue such as in the case of tumors etc., to stop bleeding or to interrupt unwanted functions, such as in the case of the so-called His bundle ablation, i.e. the interruption of atrioventricular conduction. Various different techniques are known. such as electrocoagulation, cryosurgery, cautery etc.

The principle of electrocoagulation is the application of electrical energy to the living tissue, whereby the energy has to be high enough to coagulate the protein and to dry up the tissue. For most surgical purposes side effects, such as vapor generation, chemical dissociation or pressure shock waves due to ac discharge are not critical. But in some specific surgical methods, such as in the case of His bundle ablation, the mentioned side effects are extremely dangerous.

It is the object of the present invention to avoid such dangerous techniques and therefore, to provide an apparatus for safer controlled tissue destruction.

According to the present invention this is achieved with an apparatus of the initially-mentioned kind in which the pulse generator is adapted to produce a series of electrical pulses having a voltage of between 500 and 3600 Volts and a duration of between 5 and 500 microseconds.

The inventors have discovered that an electric current of sufficiently high current density applied to living tissue for a sufficient length of time will cause localised cellular death. However, the inventors have found that application of a single pulse of sufficient current density and duration to living tissue produces unwanted and harmful side-effects.

For example, in the case of His bundle ablation, a total pulse duration of 100 to 200 micro secs has been found to be necessary to cause the required tissue damage. In such a case, the small electrode is in contact not only with muscle tissue but also with blood. Electrical power dissipated in the blood serves no practical purpose but can lead to over-heating in the blood around the electrode. This over-heating can lead to sparking and the formation of bubbles in the blood. These bubbles expand and collapse rapidly, producing harmful large pressure waves.

The inventors have discovered that these side-effects can be reduced or avoided by breaking up the single pulse into a series of short pulses separated by waiting periods. These waiting periods allow built-up energy, induced by the previous short pulse, to be at least partially dissipated before the next short pulse is generated. In this way, the harmful side-effects are reduced or avoided and the required cellular damage is still achieved.

For example, in the case of His bundle ablation, cellular damage may be achieved without harmful side-effects by application of approximately sixteen pulses of ten micro secs each, preferably with waiting periods of approximately five hundred micro secs interspersing the pulses.

It is preferred that pulses of relatively flat-topped shape be used, for example of approximately square or arch shape waveform. Use of pulses of this shape mean that, for the majority of the pulse, the pulse is at maximum current value, reaching that value quickly and decaying quickly. Such pulses are more efficient, having a higher average current value, than other, less preferred pulse shapes such as an exponentially decaying pulse. Furthermore, the preferred pulses decay rapidly, allowing a series of pulses to be generated relatively closely spaced together.

In the following a preferred embodiment of the invention is described making reference to the accompanying drawings. It is shown in

Fig.1 a schematic representation of a preferred embodiment of the apparatus applied to patient

Fig.2 a block diagram of the pulse generator

Fig.3 a circuit diagram of the power supply part of the pulse generator

Fig.4 a schematic representation of the various parts of the control logic pulse generator

Fig.5 a block diagram of the control part of the control logic pulse generator

Fig.6 a circuit diagram of the arm/disarm section of the control logic pulse generator

Fig.7 a circuit diagram of the fire/sync section of the control logic pulse generator

Fig.8 a block diagram of the self test generator of the control logic pulse generator

Fig.9 a block diagram of the sync indicator of the control logic pulse generator

Fig.10 a block diagram of the selection part of the control logic pulse generator

Fig.11 a block diagram of the pulse width selector of the selection part

Fig.12 a block diagram of the space select circuit of the selection part

Fig.13 a block diagram of the number select circuit of the selection part

Fig.14 a block diagram of the ECG detector/synchroniser part of the pulse generator

Fig.15 a circuit diagram of the ECG detector

Fig.16 a circuit diagram of the ECG filter

Fig.17 a circuit diagram of the ECG synchroniser

Fig.18 a block diagram of the high voltage generator part of the pulse generator

Fig.19 a circuit diagram of the level shifter

Fig.20 a circuit diagram of one switch section

Fig.21 a circuit diagram of the generator charge status section

Fig.22 a circuit diagram of the output voltage detector

Fig.23 a circuit diagram of the energy dump section

Fig.24 a circuit diagram of the over current detector

Fig.25 a block diagram of the safety supervisory circuit of the pulse generator

Fig.26 a circuit diagram of the electrode fault circuit

Fig.27 a circuit diagram of the system error detection

Fig.28 a circuit diagram of the charge retained error generator

Fig.29 a schematic representation of one embodiment of a tissue electrode

Fig.30 a schematic representation of another embodiment of a tissue electrode.

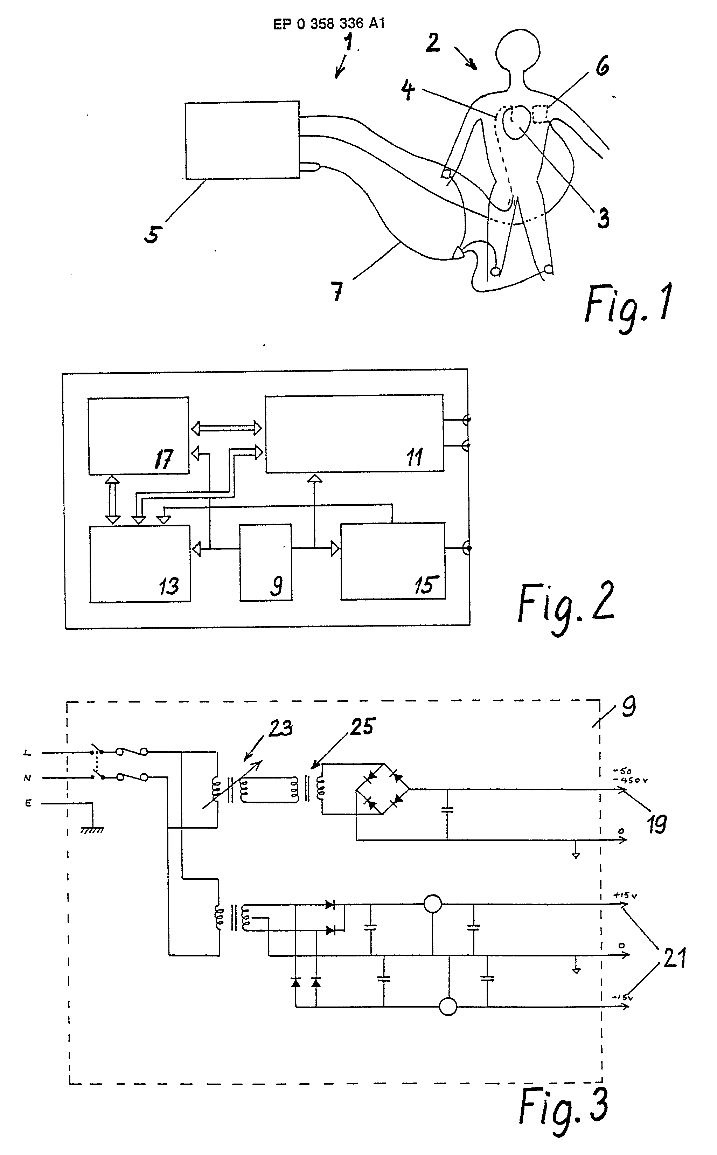

Figure 1 shows a schematic representation of one embodiment of the apparatus 1. This arrangement shows its application to a patient 2 to produce a controlled lesion in the heart 3 in vicinity of the His bundle. The method used is a unipolar method which may be preferred in some circumstances to a bipolar method.

An electrode 4 has gained access to the required site in the right side of the heart 3 by a puncture in the femoral vein. It is connected to one output of a pulse generator 5. The return path for the current from the pulse generator 5 is via an extracorporal large area electrode 6 which is connected to the second output of the pulse generator 5. For safe operation it is expected that the energy should be applied at an appropriate time in the electrical cycle of the heart. Using a surface electrode connected electrocardiographic lead 7, the generator may be suitably synchronised for a safe application of the impulses.

The pulse generator 5 shown as a block diagram in Fig.2, consists of five parts, a power supply 9 a high voltage generator 11, a control logic pulse generator 13, an ECG synchroniser 15 and a safety supervisory circuit 17.

The control logic pulse generator 13 allows the selection of pulses from 10 μs to 500 μs with spacings of 1 to 99 times the selected pulse width. Trains of pulses from 1 to 999 may be selected. These low voltage pulses are converted into the therapeutic high voltage pulses by the high voltage generator 11. This is an eight stage multiplier which can produce pulses up to 3600V and can deliver currents in excess of 36A. The rise and fall times are approximately 2 μs and are almost linear. This is achieved by the sequential switching of the eight stages of the multiplier. The advantages of the switching circuit are

(a) the very high voltages required for the therapy are not present until they are required,

(b) a modest voltage of 450V is adequate to power the device,

(c) the output voltage may be changed or selected by the relatively safe adjustment of this lower voltage and

(d) high speed readily available solid state devices may be used in its construction.

The power supply part 9 is shown in Fig.3 and has an output 19 providing a manually variable 60 to 450V D.C. voltage for the high voltage generator and a further output 21 for supply of ±15V D.C. power for the analogue and digital control circuits. The design is conventional and employs e.g. variable transformer 23 and a step up isolating transformer 25. As an alternative it may use a switched mode power supply (not shown) for the 450V requirement.

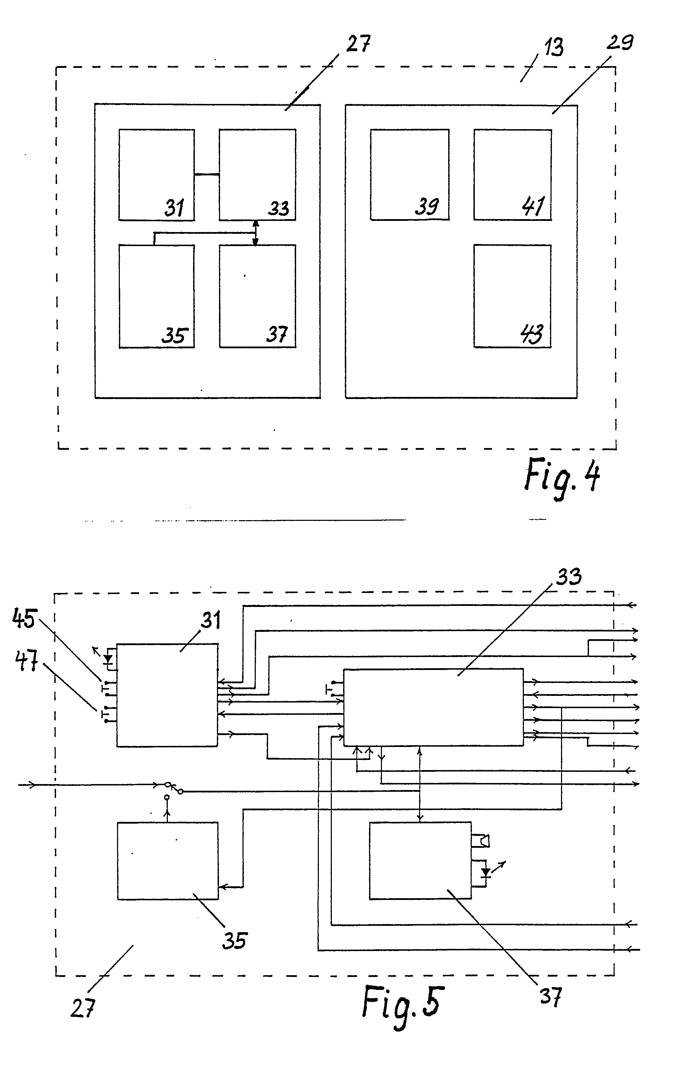

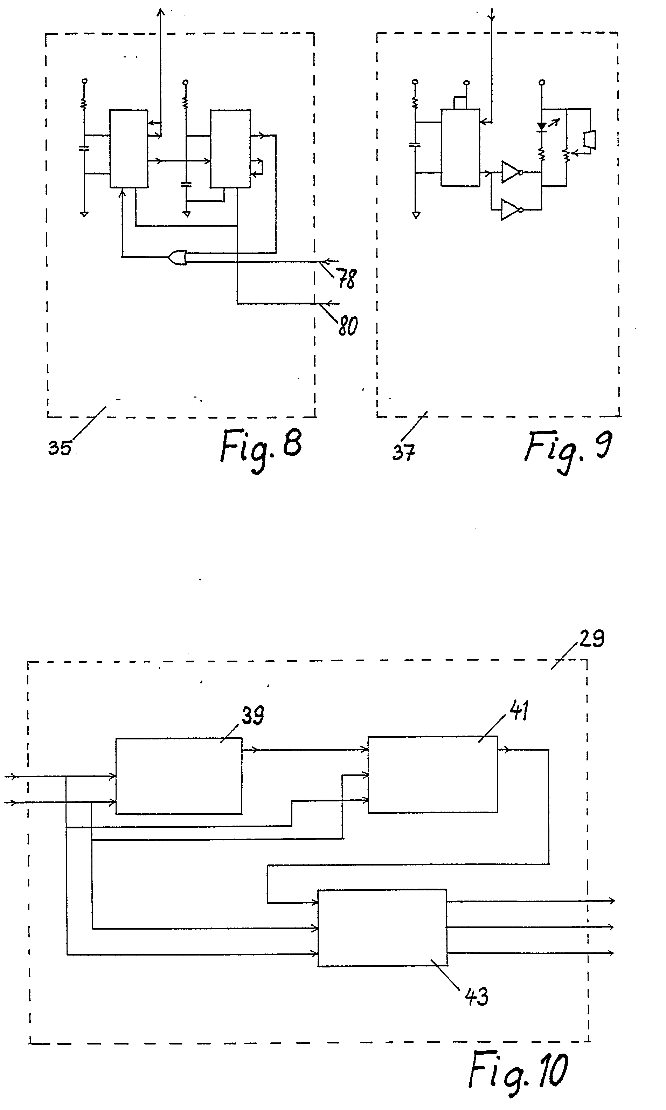

The control logic pulse generator 13 shown as a block diagram in Fig.4 produces the desired pulses to activate the high voltage generator 11. For convenience CMOS logic has been used, but almost any logic family may be used. The pulse parameter controls allow the selection of pulse length, number and spacing. It consists internally of two parts i.e. a control part 27 shown in Fig.5 and a selecting part 29 shown in Fig.10.

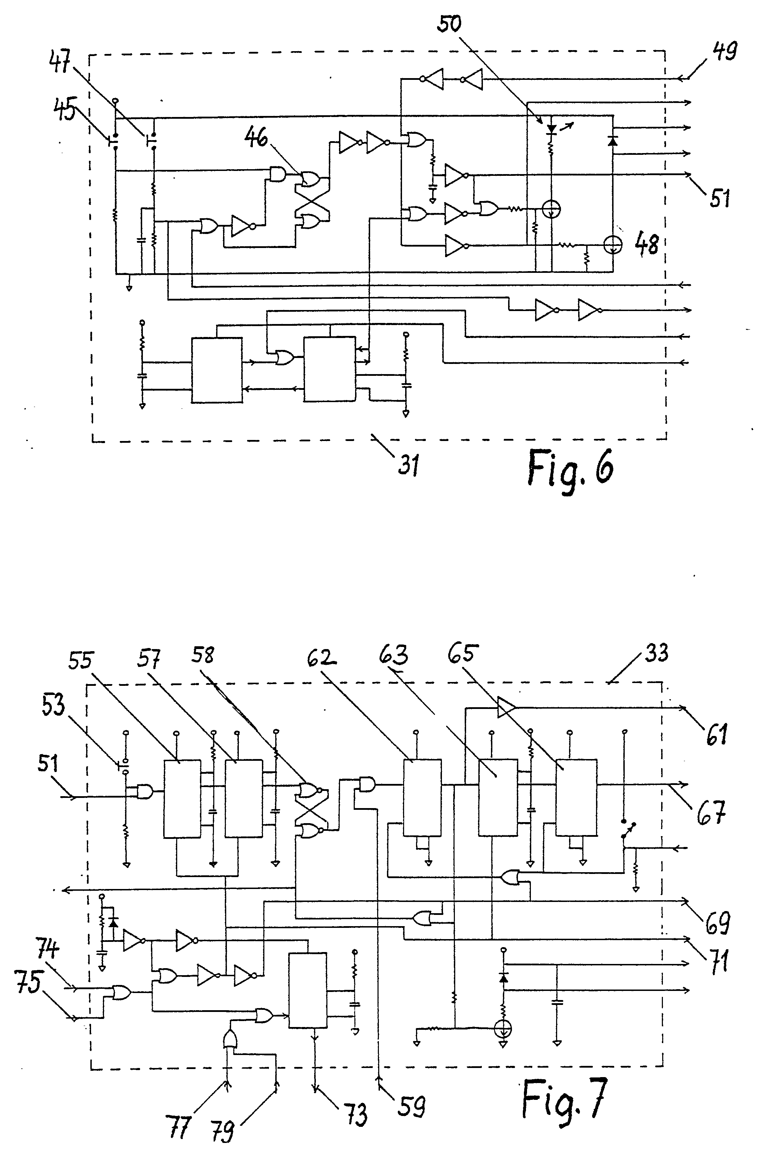

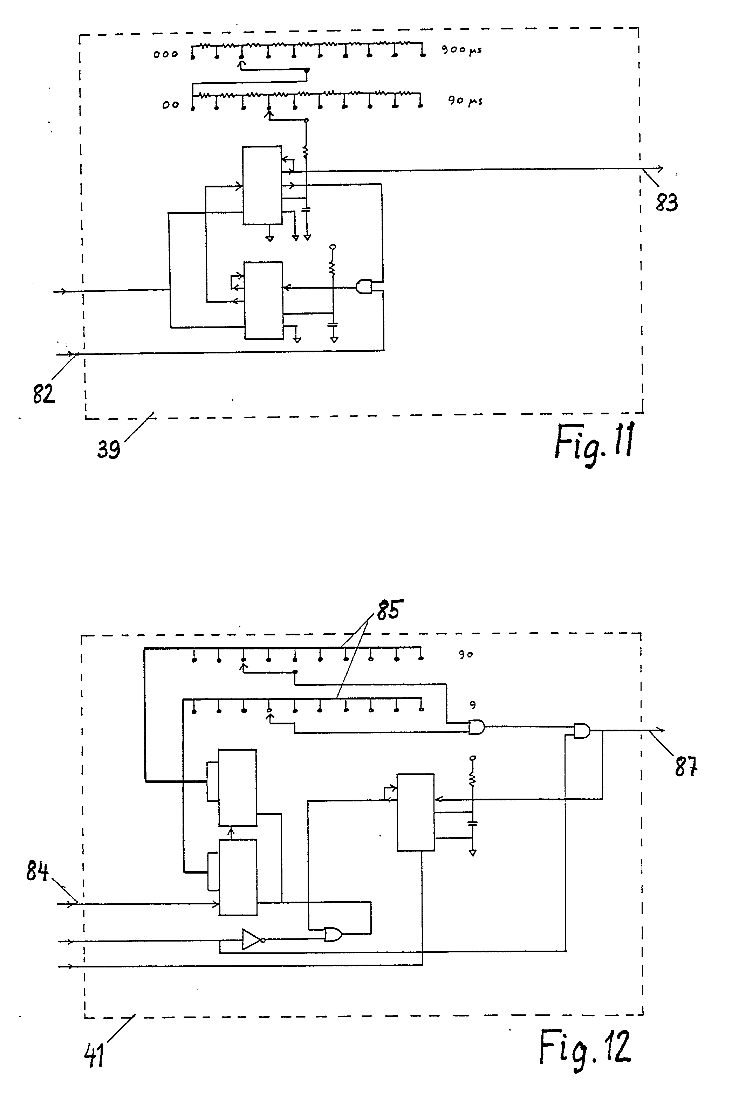

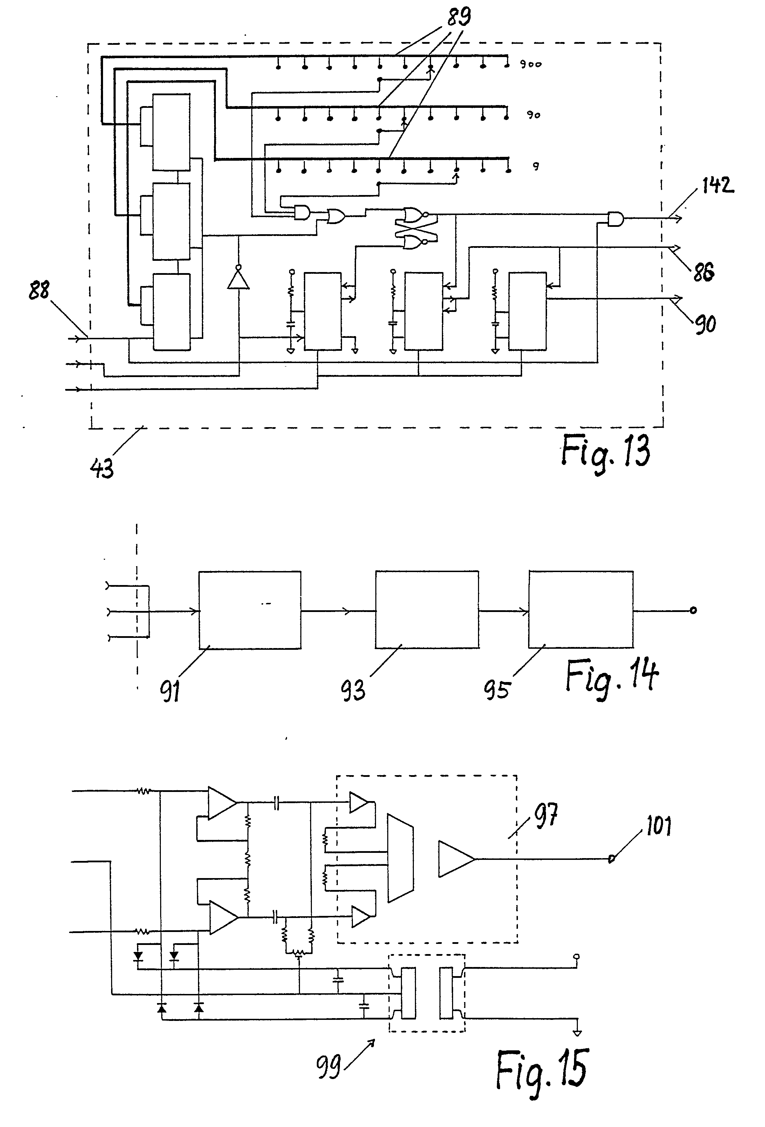

The control part 27 has been further sub-divided into four sections, i.e. an arm/disarm section 31 shown in Fig.6, a fire/sync section 33 shown in Fig.7, a self test generator 35 shown in Fig.8 and a sync indicator 37, shown in Fig.9. As shown in Fig.10, the selecting part 29 consists of a pulse width select section 39 shown in Fig.11, a space select 41 shown in Fig.12 and a number select 13 shown in Fig.13.

The operation of the control logic pulse generator 13 is as follows: The actuation of the arm firing button 45 sets a latch 46 provided that the disarm circuit 47 has not been simultaneously operated. This latch immediately closes a charge relay 48 in order to energise the high voltage generator circuit 11. A front panel light 50 associated with the arm indicator commences to flash. A signal from the high voltage generator board 11, arrives through an input 49 and indicates to the arm/disarm section 31 when the generator is fully charged and ready to operate. When this occurs the arm light remains illuminated continuously. In addition a signal is provided at an output 51 to be sent to the fire/sync section 33 which enables the firing circuit to operate. If a disarm operation is conducted at this time the charge relay is opened and the energy on the high voltage generator 11 is safety dumped into an internal load.

The fire/sync section 33 operates as follows. On receipt of a fire enable signal from the arm/disarm section 31 via lead 51 the firing switch 53 on the front panel is enabled. Operation of this control initiates the firing sequence. After passing through two monostables 55, 57 in order to remove the possibility of multiple firing pulses, a second latch 58 is set. The circuit then waits for a synchronisation pulse from the ECG synchroniser section 15 arriving via lead 59. When this is received a third latch 62 is set. This latch initiates three operations, it closes the delivery relay through lead 61, resets the arm/disarm section and it actuates a delay monostable 63 which has a period of approximately 30 ms. This is to allow sufficient time for the delivery relay to actuate and any contact bounce to have decayed. The output from this monostable then sets a fourth latch 65 which generates a signal to the selecting part 29 via lead 67.

The fire/sync section 33 also contains the circuitry for setting the instrument into the correct mode by generating signals at a power up reset output 69 and the inverse output 71 thereof. It also contains the main part of the circuitry to provide an energy dump 73 in order to make the equipment safe after the delivery of the pulse train and to respond to certain types of errors. It accepts signals at inputs for fault reset 74, system error 75, delayed stop 77 and disarm dump 79. These will be referred to in later sections.

The self test sync generator 35 shown in detail in Fig.8 is additional circuitry which allows the servicing and the setting up of the equipment without the need of an electrocardiogram. It is not essential for the correct operation of the instrument for therapeutic use. At its input 78 it receives the signal from output 69 of fire/sync section 33 and at its input 80 the inverse signal from output 71. The output from the self test sync generator may be input to the fire/sync section via an internal switch.

The sync indicator 37 shown in detail in Fig.9 provides audible and visual indication that a suitable ECG has been detected and a synchronisation signal is available for the equipment to operate. It is of a conventional design.

The output 67 from fourth latch 65 passes on to input 82 of the pulse width selection section 39 (Fig.11). This is a conventional pulse generator where the length of pulse is selectable from 10 $\mu$s to 500 $\mu$s. The output 83 from this section passes on to an input 84 of the space select section 41 shown in detail in Fig.12. A front panel control 85 connected to this part of the circuitry allows the selection of space intervals between the pulses of 1 to 99 times the selected pulse width. The output 87 from this section is connected to an input 88 of the number select circuit 43 shown in detail in Fig.13. The section designated number select 43 is connected to front panel controls 89 which allow the choice of between 1 and 999 pulses in a discrete pulse train from its output 142. At the end of selected number of pulses two signals are generated. A stop signal at output 88 and 30 milliseconds later a delayed stop signal at output 90. The stop signal is used by the fire/sync circuit 33, to reset the latches. This de-energises the delivery relay thus disconnecting the subject from the internal circuitry of the pulse generator. The delayed stop signal is used to initiate an energy dump and remove the residual energy in the high voltage generator.

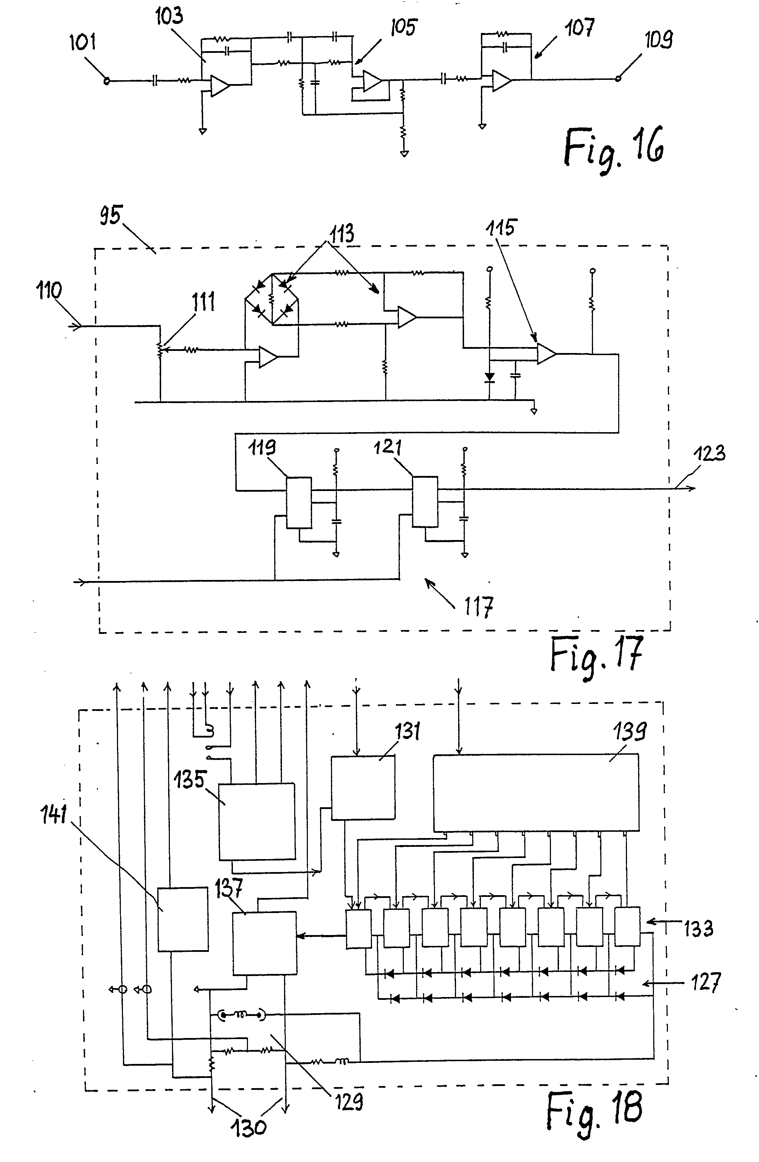

The part of the instrument which has been designated ECG detector/synchroniser 15 consists of three parts in itself shown schematically in Fig.14. The internal parts have been called the ECG detector 91 shown in Fig.15, ECG filter 93 shown in Fig.16 and ECG synchroniser 95 shown in Fig.17. The ECG detector section 91 shown in detail in Fig.15 is a conventional isolated pre-amplifier. It has been realised using an optoisolator 97 with additional pre amplification before the isolation barrier. It has over load protection and contains an isolated power supply 99. The output 101 from this section may be designated wide band electrocardiogram. The wide band ECG is processed by the ECG filter section 93. This is shown in detail in Fig.16 and consists of a preliminay band pass filter circuit 103 and followed by a line frequency notch filter 105. The output from the notch filter is passed through a further band limiter 107 in order to reduce commonly encountered bioelectric artfacts which may be present on the ECG, for example, respiratory baseline wander and somatic tremor. The signal output 109 may be designated filtered electrocardiogram. This signal is fed to input 110 of the ECG synchroniser section 95 shown in detail in Fig.17. After passing through a potentiometer 111, allowing manual adjustment of the level to the ECG synchroniser, the signal is fed to a precision rectifier 113 to allow for either polarity of the ECG R wave. A comparator 115 detects the leading edge of the R-wave. However, in some circumstances the T-wave may be excessively large and these may cause an incorrect synchronisation of the therapeutic impulses. To guard against this possibility a paralysis circuit 117 is added which comprise two sequential monostables 119, 121. The output 123 from this section is a very short pulse which is passed to the fire/sync circuit 33. Although manual adjustment is used in the present embodiment this may be an automatic function in other embodiments.

The high voltage generator section 11 shown in Fig.18 operates by charging eight capacitors in the switch sections 133 in parallel, using diodes 127 and an inductor 129 to complete the circuit. A high voltage pulse is produced at output 130 by switching the capacitors in series. Therefore, if 450V is stored on each capacitor an output of 3600V should be available from the high voltage generator.

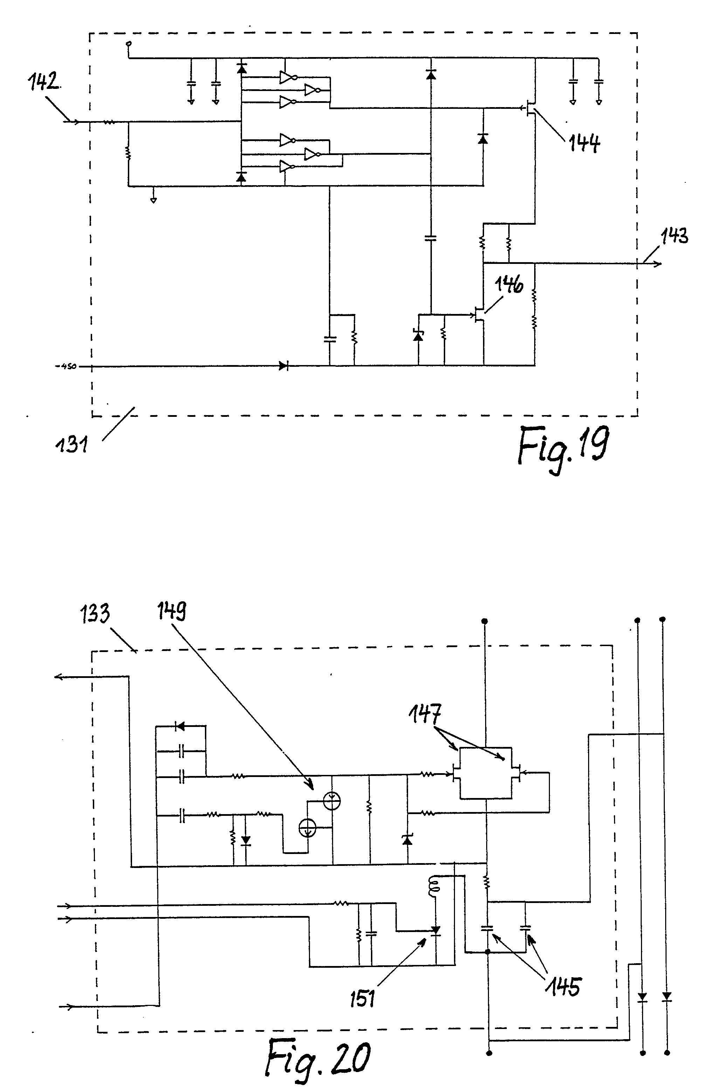

It consists of a level shifter 131, eight switch sections 133, a generator charge section 135, an output voltage detector section 137, an energy dump section 139 and an over current detector 141. The level shifter section 131 shown in detail in Fig.19 accepts at its input 142 the pulse from the number select section and converts this into a pulse with an amplitude equal to the negative supply voltage to the high voltage generator 11. Discrete power MOS-FET 114, 146 devices are used to provide active turn on and active turn off for the level shifter output 143 which may be designated HV drive.

There are eight switch sections 133 which are all identical. Each consists of a storage capacitor 145, a pair of power MOSFET transistors 147, a drive circuit 149 for the gates of these transistors, and a thyristor energy dump device 151. The output from the level shifter initiates the turn on of the lowest switch section. When this section is fully turned on, which takes approximately 250 ns, it initiates the turn on of the second section. When the second section is fully on this turns on the third section and so on until all eight sections are turned on. The output is essentially a staircase, or a linear rise in voltage over approximately 2 μs. The turn off of the high voltage generating sections follows a similar course, whereby the lowest section turns off first which initiates the turn off of subsequent stages. Again a linear fall in output voltage is produced.

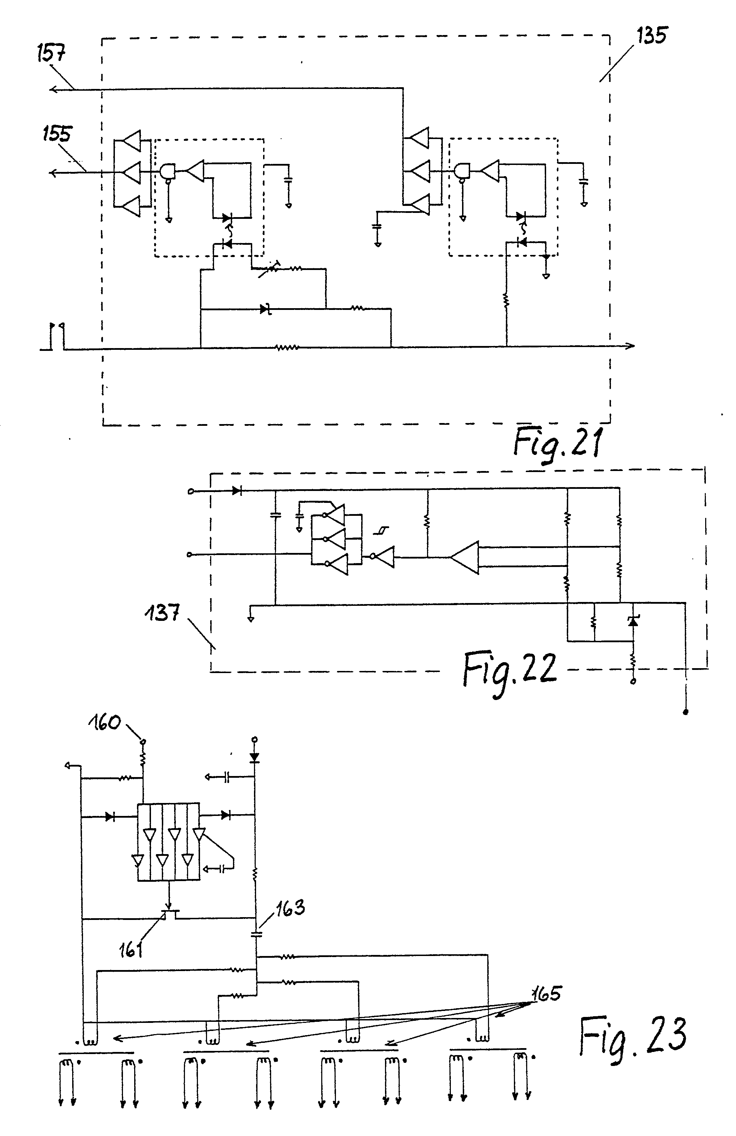

When the charge relay is energised by the arm/disarm circuit, current flows from high voltage power supply to charge the capacitors 145 to the predetermined level. When the charging current has fallen to a relatively low value, indicating that the capacitors are fully charged, an output 155 from the generator charge status section 135 shown in detail in Fig.21 goes low. This is used to enable the firing button on the fire/sync section. The generator charge status 135 also provides an output 157 called capacitor charged, which is used by the safety supervisory circuit section to indicate that under certain circumstances a fault has occurred in the instrument.

The output voltage detector 137 shown in detail in Fig.22 generates an output 159 which is also used by the safety supervisory circuit.

The energy dump section 139 shown in detail in Fig.23 is used to actuate the thyristors on the high voltage generator board in order to dissipate the energy stored in the capacitors in the switching sections. This occurs under three conditions, (1) at the end of the pulse sequence, (2) if a fault is detected or (3) if the instrument is disarmed without firing. The energy dump section accepts at its input 160 a signal from the fire/sync board designated energy dump and, using a conventional FET switch 161 and capacitor 163, generates a pulse in four pulse transformers 165, which are connected to the gate cathode circuits of the thyristors.

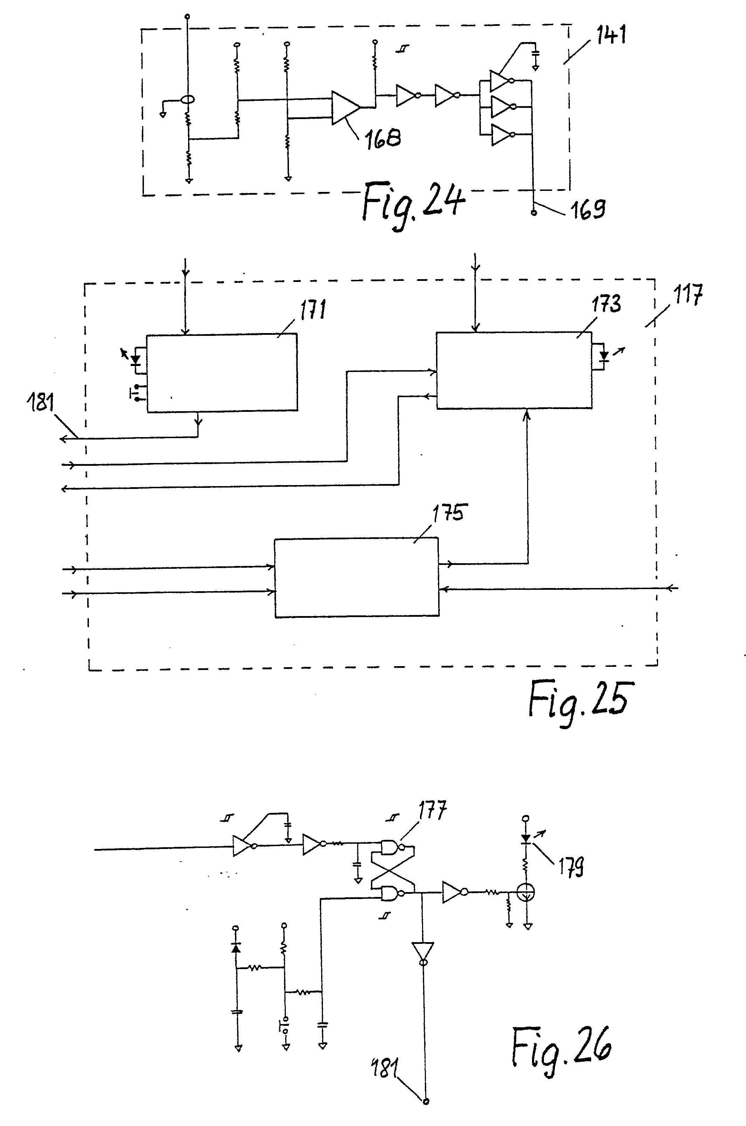

A 0.5 ohm resistor 167 (shown in Fig.18) in the return path from the subject generates a voltage proportional to the current flowing between the electrodes. The over current detector circuit 141 shown in detail in Fig.24 examines this voltage and, if it exceeds the equivalent of 50 Amps of current flow, will produce, via a comparator 168, an error signal at its output 169 designated over current. This signal is used by the safety supervisory circuit, see below.

The safety supervisory circuit 17 shown in Fig.25 consists of three parts. These have been designated electrode fault 171, system error detector 173 and charge retained error detector 175. The electrode fault section 171 shown in detail in Fig.26 accepts an over current logic level signal 169 which will then actuate an internal latch 177. This illuminates a front panel indicator 179 and generates a fault reset logic level at its output 181 for the fire/sync section. If this occurs, then it is necessary to manually reset the electrode fault circuit. No pulses can be deliveed until this has been done. This is intended to make the operator aware that an excessive current has flowed and it is necessary to check the lead connections external to the pulse generator.

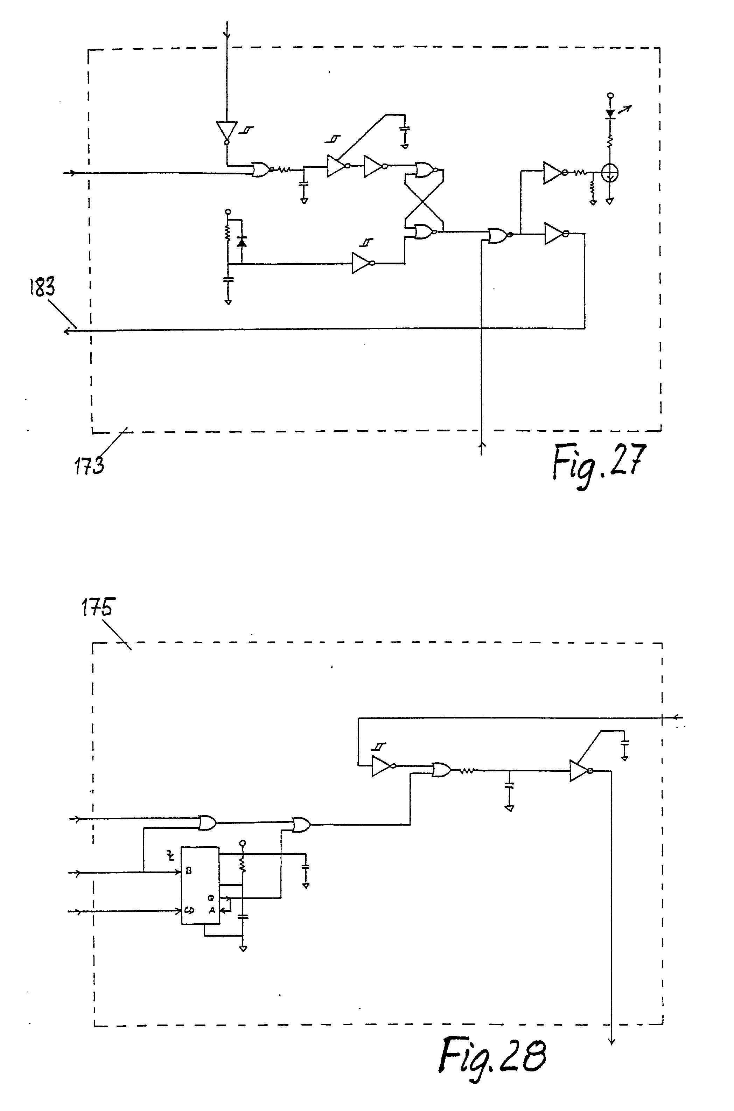

The system error detector 173 shown in detail in Fig.27, is used to detect a fault in the high voltage generator. If the output pulse is significantly longer than the control logic pulse generator input pulse to the level shifter then this circuit is activated. It produces a system error signal at its output 183 for the fire/sync board and there is no way of resetting the circuit, without turning the instrument off then on again thereby actuating a power up reset operation. The system error output can also be produced if the charge retained error generator also detects a fault. There are various failure modes which could produce charge on the capacitors when it should not be there.

The charge retained error generator 175 shown in detail in Fig.28 ignores the capacitor charged signal during the closure of the charge relay or the delivery relay and for 30 ms later. One condition which could occur would be an incorrect or absence of the firing of the energy dump thyristors. Under these conditions the instrument is completely disabled.

The electrode used in this cardiological procedures with the low energy generator was constructed from a commercially available temporary pacing lead. These leads in their standard form ae usually multipolar with individual exposed surface electrode areas of not more than approximately 12 sq mm. They are unsuitable for immediate use in this application for three reasons. First, the electrical insulation between poles is often not adequate for the short duration, high voltage, therapeutic pulses when applied to energise only a single pole of a multipole lead. The energy is often delivered to the tissue from the unenergised exposed electrodes due to electrical insulation breakdown. The second problem is the surface area of the activated electrode. To obtain satisfactory electrical impedance characteristics of the electrode/tissue interaction for the low energy impulses, the surface area is too small. Finally, the exposed electrode geometry is unsuitable. It produces a sub-optimal field distribution around the electrode.

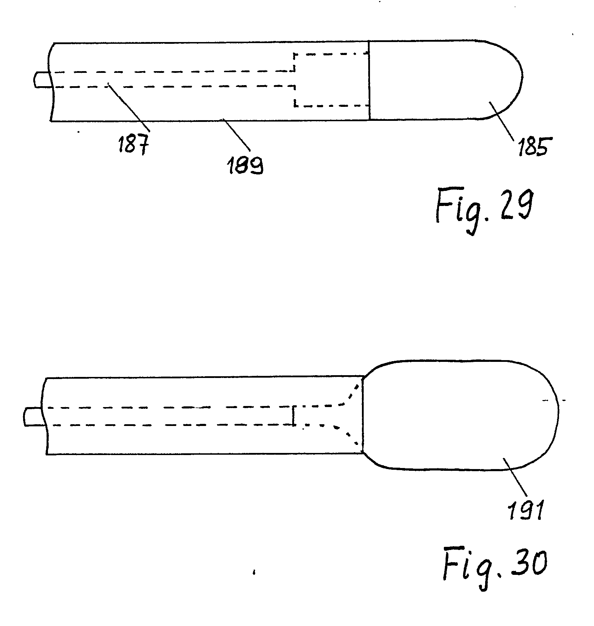

An electrode, which has been constructed and evaluated, is shown in Fig.29. It has a cylindrical head 185 with a hemi-spherical tip. A conductor 187 for electrical connection is covered by an insulating layer 189. The lead impedance was less than 2 ohms and the surface area of the exposed electrode was approximately 25 sq.mm. This has proved to be more satisfactory than the regular pacing leads in clinical practice. A second electrode, shown in Fig.30, has been constructed which has optimised geometry and a higher surface area. This is achieved by an increased head portion 191. It has been evaluated and has shown a further improvement in electrical/tissue characteristics. It has allowed the delivery of the required current for cardiac use at lower delivered energy.

The method and device hereinbefore described exhibit the following particular advantages over conventional techniques:

- A controlled lesion may be produced with the electrode and pulse generator which does not require regular surgery.
- The lesion size may be controlled by the selection of a suitable output from the pulse generator.
- The potentially harmful effects of other electrical techniques, e.g., defibrillators, in the form of electrode arcing and mechanical pressure effects are completely eliminated with this technique.
- The electrical stresses induced by instantaneous voltage rises associated with simple capacitor discharges have been eliminated by using a controlled pulse rise time.
- The excessive energy delivered by other techniques during the extended pulse tail have been eliminated by using a controlled pulse fall time.
- The application of a constant voltage for a preset time allows more control over the lesion boundary and extent.
- The technique has been demonstrated to be safe for use with procedures requiring the cardiological therapy of His bundle block. It is therefore the only safe method presently available for accessory pathway, via the coronary sinus, and ventricular tachycardia foci ablation.
- The technique may be used for the controlled therapeutic destruction of other living tissue.
- A high voltage and a high current capability are available from a much lowe and, hence, safer energising voltage.
- The high voltage and high current amplitudes are controllable over a wide range.
- The pulse width, spacing and number of pulses may be easily selected and controlled.
- The pulse rise and fall times are almost linear and controlled by the circuit design.
- The therapeutic pulse or pulses may be synchronised, for cardiac use, to occur during the safe period in the cardiac electrical cycle.
- The pulse generator may be used in unipolar or bipolar mode.
- The electrode area is optimised for the minimum energy delivery consistant with no arcing and mechanical pressure effects.
- The electrode geometry is optimised for the minimum energy delivery consistant with no arcing and mechanical pressure effects.
- The electrode area and geometry are optimised for the minimum voltage requied to achieve an adequate current flow. Hence the lowest dielectric stress on the insulation of the probe and the lowest risk t the operator and patient.

The features disclosed in the foregoing description, in the following claims and/or in the accompanying drawings may, both separately and in any combination thereof, be material for realising the invention in diverse forms thereof.

## Claims

1. Apparatus for effecting controlled destruction of limited areas of living tissue by applying electrical pulses of high current to the tissue, having an electrical pulse generator, a probe with an electrode to be placed close to or in the tissue area to be treated and a second electrode for placement in contact with the patient to complete the electrical circuit, characterized by the part that the pulse generator is adapted to produce a series of electrical pulses having a voltage of between 500 and 3600 Volts and a duration of between 5 and 500 microseconds.

2. Apparatus according to claim 1 in which the second electrode is a large area skin electrode.

3. Apparatus according to claim 1 in which the second electrode is a tissue electrode like the first electrode.

4. Apparatus according to claim 1 wherein the pulse generator comprises a high voltage generator having a multistage multiplier.

5. Apparatus according to claim 1 in which the pulse generator comprises means for soluting pulses of 10 µs to 1 ms duration.

6. Apparatus according to claim 1 in which the pulse generator comprises means for soluting pulse spacings of between 1 and 11 times the selected pulse width.

7. Apparatus according to claim 1 in which the pulse generator contains energy dump means to dissipate stored energy.

8. A method of effecting controlled destruction of limited areas of living tissue by applying electrical pulses of high current to the tissue, which method comprises using an apparatus having an electrical pulse generator, a probe with an electrode to be placed close to or in the tissue area to be treated and a second electrode for placement in contact with the patient to complete the electrical circuit, characterized in that the pulse generator produces a series of electrical pulses having a voltage of between 500 and 3600 Volts and a duration of between 5 and 500 microseconds.

Fig.1

Fig.2

Fig.3

Fig.4

Fig.5

Fig. 6

Fig. 7

EP 0 358 336 A1

Fig. 8

35

78

80

Fig. 9

37

Fig. 10

29

39

41

43

ooo                                                    900 µs

oo                                                     90 µs

83

82

39

*Fig. 11*

85

90

9

87

84

41

*Fig. 12*

Fig. 13

Fig. 14

Fig. 15

Fig. 16

Fig. 17

Fig. 18

Fig.19

Fig.20

*Fig.21*

*Fig.22*

*Fig.23*

Fig. 24

Fig. 25

Fig. 26

183

173

*Fig.27*

175

*Fig.28*

187    189    185

Fig. 29

191

Fig. 30

## DOCUMENTS CONSIDERED TO BE RELEVANT

| Category | Citation of document with indication, where appropriate, of relevant passages | Relevant to claim | CLASSIFICATION OF THE APPLICATION (Int. Cl.X) 5 |
|---|---|---|---|
| Y | WO - A1 - 87/04 081 (LICENCIA) * Claims; fig. * | 1,2,3,8 | A 61 B 17/36 A 61 N 1/32 |
| Y | US - A - 4 324 253 (GREENE) * Abstract; claim 6 * | 1,2,3,8 | |
| A | DE - A1 - 3 716 816 (PHYSIOMED) * Abstract; claims 1,2 * | 1,8 | |
| A | EP - A2 - 0 287 368 (NARULA) | | |

TECHNICAL FIELDS
SEARCHED (Int. Cl.X) 5

A 61 B
A 61 N

The present search report has been drawn up for all claims

| Place of search | Date of completion of the search | Examiner |
|---|---|---|
| VIENNA | 19-12-1989 | NARDAI |